# EUROPEAN PATENT APPLICATION

(11) **EP 1 247 456 A2**
(43) Date of publication of application: **09.10.2002**
(21) Application number: 02251211.5
(22) Date of filing: 22.02.2002
(51) Int. Cl.: A23K 1/18, A61K 9/00

(54) **Palatable pharmaceutical compositions for companion animals**

(30) Priority: 28.02.2001 US 272022 P
(71) Applicant: Pfizer Products Inc., Groton, Connecticut 06340 (US)
(72) Inventor: Kasraian, Kasra, Pfizer Global Res. & Development, Groton, Connecticut 06340 (US); Thombre, Avinash G., Pfizer Global Res.&Developmt., Groton, Connecticut 06340 (US)
(74) Representative: Wood, David John

(57) **Abstract**

The present invention is directed to pharmaceutical compositions, which are palatable to companion animals wherein the pharmaceutical composition comprises pharmaceutically effective amounts of pharmaceutically active agents and palatability improving agents.

## Description

### Field of the Invention

The present invention relates to novel palatable pharmaceutical compositions that can be administered orally to companion animals. In particular, the present invention relates to palatable, pharmaceutically active dosage forms containing palatability improving agents that can be orally administered to companion animals, including, for example, dogs and cats.

### Background of the Invention

Oral administration of medicaments to animals is frequently associated with great difficulties, due to the reluctance of the animals to ingest tablets, pills or medicated feed, especially if these drugs have an unpleasant taste, e.g., bitter flavor. The medicament when administered orally, for example, in a tablet form, even when combined with feed, is frequently not accepted, and the desired oral treatment either cannot be effected or can only be accomplished utilizing force, but only to a restricted and thus usually insufficient and inconsistent extent.

In companion animals, such as, for example, dogs and cats, medicated feed additives are typically restricted to neutral-tasting additives for nutrition and prophylaxis, such as vitamins, trace elements and the like, since medicaments which have an unpleasant taste, especially in an effective single dose, are not voluntarily ingested by the animal.

US Patent No. 4,283,400, issued to Von Bittera, et al. describe the use of medicated animal feeds comprising a pharmaceutically active compound and liver meal as a product isolated and extracted by drying and grinding the fresh liver of warm blooded land animals as a formulation auxiliary.

In general, treats and premium canned food products can be considered to be highly palatable. Both the treats and the premium canned food products for dogs as well as cats are generally meat based. The treats are relatively large in size and have interesting or attractive shapes and textures. The moist and semi-moist canned food preparations are more palatable than the dry pet food rations. Frequently, palatable compositions for dogs and cats are prepared from slaughterhouse waste. Naturally derived meat-based flavorings, such as liver extracts, meat-based fats and animal by-products, may vary in composition on a batch to batch basis, and therefore lack consistency with respect to product composition, making such flavorings unacceptable for use in pharmaceutical formulations. In addition, such products may present added difficulties in obtaining regulatory approval. Moreover, the use of naturally derived meat-based flavorings cannot assure adequate quality control of microbiological content, such as the presence of various pathogens, including various viruses and bacteria, such as, for example, TSE (transmittable spongiform enephalopathy).

The taste preferences of dogs and cats are well established and it is known that cats prefer fish and commercial cat food to rats. Dogs prefer beef, pork and lamb to chicken, liver and horsemeat and strongly prefer meat to cereal diets. They prefer canned meat to fresh meat, ground meat to cubed meat and cooked meat to raw meat. Canned or semi-moist preparations are preferred to dry ones. (Katherine A. Houpt and Sharon L. Smith, Taste preferences and their relation to obesity in dogs and cats, Can. Vet. J., 22:77-81, 1981.

The natural preferences of dogs and cats tend to be complex mixtures of flavors. There is a need to have simplified palatability-enhancing formulations that are effective in dogs as well as cats. Such a formulation would simplify stability testing and quality monitoring since testing would need to only be performed on a single formulation.

The determination of the palatability of a dosage form should be conducted in a uniform and consistent manner. In the past, claims made with regard to palatability have not been based on a standardized test. A standardized palatability test based on acceptance and preference would allow for a comparison and the ranking of flavors relative to each other and the selection of optimum flavors and formulations for palatable dosage forms.

There is also a need to manufacture dosage forms using technology that is well established. For the manufacture of tablets, a blend of active drug and excipients can be prepared and compressed using conventional rotary tabletting machines. In such operations, a blend requires acceptable compressibility and flow characteristics.

As a result of these problems, it is desirable to utilize palatability improving agents in veterinary pharmaceuticals that are not meat-based or derived from meats such as beef, lamb, pig, and the like, or fish or poultry.

Another known approach is the addition of yeast to a pharmaceutically active compound in order to mask the taste. US Patent No. 5,824,336, issued to Jans, et al describes the addition of large amounts of brewer's yeast to flubendazole to mask the taste of the pharmaceutically active compound. More specifically, it describes brewed yeast present in an amount of from 40% to 70% by weight of the pharmaceutical composition. However, Jans et al. do not describe or suggest the addition of yeast in significantly smaller amounts for improving palatability.

US Patent No. 4,118,512 issued to Eichelberg describes the use of yeast hydrosylate to enhance the palatability of oral ingests, such as animal food or orally administered animal medicaments. It describes that the yeast hydrosylate may be combined with the orally administered animal medicaments, with the yeast hydrolysate being present in amounts ranging from at least 2% to about 99% by weight and more preferably from about 7% to about 99% by weight. It further describes that the yeast hydrolysate can be used in a coating wherein the amount added ranges from about 0.5% to about 99%.

However, Eichelberg, et al. do not suggest any upper limit for the addition of yeast to ingest. The present inventors, however, have found that yeast provides for palatable pharmaceutical compositions, even at low concentrations, and furthermore, that there is an upper limit to the amount of yeast that may be added, above which the yeast will not significantly improve the palatability of the off-tasting or bitter pharmaceutically active agent.

It is advantageous to use the least amount necessary of palatability improving agents in order to minimize expenses for and maximize efficient production of palatable pharmaceutical compositions. Moreover, it is desirable to utilize such palatability improving agents in least necessary amounts in order to minimize possible toxic effects.

From the above, it would be expected that in order to make something palatable to dogs and cats, it should contain meat, it should be moist (i.e. high water content), it should be soft, and it should have an interesting shape and texture. It is unexpected that a pillable dosage form that is small, hard, with a standard tablet shape, dry and free of meat can be made palatable. Also, given the differences in taste preferences between dogs and cats, it would be unexpected that a single and identical or similar dosage form could be made palatable to both dogs and cats.

In view of the above deficiencies of pharmaceuticals available for oral administration to animals, there is a need for a palatable pharmaceutical composition in which unpleasant, usually bitter-tasting, or neutral-tasting, pharmaceutically active agents can be administered orally to animals in a consistent and precisely dosed form and which are voluntarily accepted or ingested by the animals.

The present inventors have found palatability improving agents, not derived from meat, fish, or poultry, which when combined with a pharmaceutically active agent, and administered orally to animals, mask the unacceptable taste of an active agent or alternatively, improves the acceptance or ingestion of a neutral-tasting pharmaceutically active agent, without altering its efficacy.

Furthermore, the present inventors have found that it is advantageous to homogeneously mix such a palatability improving agent with a pharmaceutically active agent. The resulting pharmaceutical composition is voluntarily accepted or ingested by an animal and can be administered in a precise dosage form of consistent composition and stability.

Except for yeast, no one has suggested the use of such palatability improving agents to be added to pharmaceutically active agents and administered to animals. Moreover, the present inventors have found the upper limit of the amount of yeast that may be added to pharmaceutically active agents. More specifically, the present inventors have found that the maximum amount should be no greater than about 25% by weight of the pharmaceutically active agent.

The present inventors have found that palatability improving agents that are pleasing to humans are not necessarily palatable to companion animals. That is, the anthropomorphic usage of additives considered palatable to humans for organoleptic acceptability does not always coincide with palatability as determined by acceptance testing of a subject animal. Also, usage of some ingredients suggested by human practice especially those of sticky or fibrous nature (in the human controlled through normal dental hygiene) are contraindicated for animals dental or gum health. Finally, for companion animals, especially dogs and cats, concerns are presented concerning some tasteful ingredients that can contribute nevertheless to halitosis.

The present inventors have discovered palatable pharmaceutical compositions in which unacceptable, unpleasant, usually bitter-tasting, or neutral-tasting, pharmaceutically active agents can be administered orally to animals in a consistent and precisely dosed form and which are voluntarily accepted or ingested by the animals. Accordingly, the palatable pharmaceutical compositions of the invention provide for superior therapeutic compliance and convenience, and greater pet owner satisfaction.

### Summary of the Invention

The present invention is related to a palatable pharmaceutical composition for oral administration to companion animals comprising a pharmaceutically effective amount of a pharmaceutically active agent in combination with a palatability improving agent and a pharmaceutically acceptable carrier, wherein the palatability improving agent is non-meat and non-fish derived, and is present in amounts sufficient to make the pharmaceutical composition palatable to the companion animal, provided that when the palatability improving agent is yeast, it is present in an amount ranging from about 2% to about 25% by weight of the palatable pharmaceutical composition.

In one aspect of the invention, the addition of the palatability improving agent to the pharmaceutically active agent provides for voluntary acceptance by the companion animal of the pharmaceutically active agent, which is acceptance is greater than or equal to about 30% voluntary acceptance, preferably greater than or equal to about 50% voluntary acceptance, and more preferably greater than or equal to about 80% or greater.

Preferably the difference between the acceptance rate of the palatable pharmaceutical composition and the pharmaceutically active agent is statistically significant at the 95% confidence level.

In another aspect of the invention, the pharmaceutically active agent is homogeneously mixed with the palatability improving agent.

In a further aspect of the invention, the palatability improving agent is selected from the group consisting of artificial egg, artificial beef, artificial poultry, artificial fish, dairy based palatability improving agent and natural herbs and spices, or a mixture thereof.

In accordance with the invention, the palatability improving agent is present in amounts sufficient to improve the palatability of the pharmaceutically active agent by masking the undesirable, e.g. bitter taste, or improving the neutral taste of the pharmaceutically active agent. Preferably the palatability improving agent is present in an amount ranging from about .025% to about 99% by weight of the pharmaceutical composition, more preferably in an amount ranging from about .075% to about 50% by weight of the pharmaceutical composition.

If the palatability improving agent is yeast or yeast hydrolysate, it is preferably present in an amount ranging from about 2% to about 25%, more preferably in an amount ranging from about 5% to about 25%.

The companion animal, to which is administered the palatability improving agent, such as yeast or yeast hydrolysate, artificial egg, artificial beef, artificial poultry, is preferably, a cat or a dog.

When the pharmaceutical composition is in the form of a tablet, preferably the water is present in an amount less than about 7.5% free or absorbed water content as measured by loss on drying, more preferably the water is present in an amount less than about 5% free or absorbed water content as measured by loss on drying.

When the pharmaceutical composition is in the form of a tablet, preferably, the hardness of the tablet is in the range of from about 2.5 kP to 25 kP.

The pharmaceutical composition of the invention is preferably a tablet, and more preferably, a hard compressed, substantially moisture-free tablet, wherein the pharmaceutically active agent is homogeneously mixed with the palatability improving agent.

The invention is further related to a pharmaceutical composition for administration to companion animals comprising a pharmaceutical effective amount of a pharmaceutically active agent mixed with a palatability improving agent and in association with a pharmaceutical carrier, said palatability improving agent being a yeast or yeast hydrolysate, or a combination thereof, said yeast or yeast hydrolysate or combination thereof being present in an amount ranging from about 2% by weight to about 25%, preferably from about 5% to about 20% by weight of the pharmaceutical composition.

### Detailed Description of the Invention

The term "companion animal" refers to domesticated animals. Companion animals exclude humans. Preferably, the animal is a mammal. Examples of companion animals include, but are not limited to, dogs, cats and horses. The preferred companion animals are dogs and cats.

The terms "palatability" means the voluntary (free choice) acceptance or ingestion of a pharmaceutical composition by companion animals, as measured by a standard palatability test, such as acceptance testing, preference testing or consumption testing. These tests are described below in the Examples.

The term "palatability improving agent", as used herein, includes any composition that alters the palatability of the pharmaceutically active agent to which it is added, and more particularly improves the palatability of the pharmaceutically active agent as measured by a standard palatability test, such as acceptance testing, preference testing or consumption testing. Preferably, the difference between the voluntary acceptance rate of the pharmaceutical composition containing the palatability improving agent and the pharmaceutically active agent without the palatability improving agent is statistically significant at the 95% confidence level.

Preferably, a palatability improving agent provides a voluntary acceptance by the companion animal of the pharmaceutically active agent which is greater than or equal to about 80% voluntary acceptance, and more preferably, about 90% voluntary acceptance as determined by the above mentioned tests.

"Acceptance" or "voluntary acceptance" means that the dosage form is voluntarily taken into the mouth of the animal. It is preferred that the animal voluntarily take the dosage form within its mouth within 10 minutes. It is more preferred that the animal voluntarily take the dosage form within its mouth within 5 minutes. Most preferred is that the animal voluntarily takes the dosage form within its mouth within 2 minutes.

"Pillable" means that the dosage form can be administered in the conventional manner by which tablets are given to companion animals so that the tablet is swallowed in a substantially intact form. This is also known as the "poke down" method.

"Friability" is a measure of tablet robustness to mechanical force. A standard tablet friablity test is given in the United States Pharmacopea, 24^{th} edition, <1216>, Tablet Friability.

"Tablet hardness" is the force required for breaking or crushing a tablet in a diametrical compression test. The test consists of placing a tablet between two anvils and applying pressure to the anvils until the tablet breaks. The force is generally measured in the units of kilopond, newton, strong cobb or pound.

In addition to being palatable, it is preferred that the dosage form should be such that it can be dosed in the conventional manner (also known as "poke-down") that is characteristic of a pillable dosage form. This is an important requirement for dosage forms that may need to be administered to animals that are too sick to accept the medication in a free choice manner or for certain animals that for some reason do not accept the dosage form by free choice on some occasions. Pillable dosage forms can be crushed or ground by the owner, caregiver, pharmacist, veterinarian so that it can be sprinkled on or mixed with food, dissolved or suspended in liquid, mixed with semisolid food products such as peanut butter or malt hairball remedies which can be administered directly or smeared onto the fur (i.e. back of a front paw) for ingestion by the animal during self grooming.

The present inventors have found that the addition of the palatability improving agent to the pharmaceutically active agent enhances or improves the palatability of the pharmaceutical composition by improving the acceptability, such as by taste or smell, of the pharmaceutically active agent, through the introduction of a highly pronounced and desirable agent, which is attractive to the animal. Thus, if the pharmaceutically active agent is unacceptable to an animal, such as when it has a bitter taste, or alternatively, when it has a neutral taste, the palatability improving agent not only masks the undesirable flavor associated with the pharmaceutically active agent but also attracts the animal to the pharmaceutically active agent so that it voluntarily ingests the pharmaceutical composition, resulting in a palatable pharmaceutical composition. By "unacceptable" is meant bitter or neutral tasting to a companion animal such as a dog, cat or horse.

The palatability improving agents of the invention are not meat-based or derived from meat. The term "meat means beef, lamb, or poultry. In addition, it is not fish-based or derived from fish. The palatability improving agents utilized in the present invention to be mixed or admixed with the pharmaceutically active agents are commercially available and generally acceptable for use in food applications.

The palatability improving agents of the present invention, include, but are not limited to, for example, dairy-based flavoring agents, a mixture of natural herbs and spices, artificial egg flavor, artificial meat flavor, artificial chicken flavor, artificial fish flavor, or yeast flavor, or a combination thereof. These are commercially available.

The dairy-based flavoring agents are those derived from milk or cheese but preferably low-fat cheeses and milk, e.g., evaporated milk or skim milk or malted milk, whey or other milk products. Alternatively the flavoring agent may be an imitation cheese (sodium capstan). Further soy or vegetable-based cheese substance may be used as the flavoring agent.

The palatability improving agents may be a mixture of natural herb and spices in combination. These natural herbs and spices include, for example, such spices as allspice, anise seed, caraway seeds, cardamon, celery seed, cinnamon, cassia, clover, coriander, cumin seed, paprika, dill seed, fennel seed, ginger, mustard seed, nutmeg, saffron, black pepper, white pepper, and the like, herbs, such as basil, bay, dilled, marjoram, oregano, rosemary, sage, savory, tarragon, tumeric and thyme.

Moreover, the palatability improving agent may include seasonings, which are dry mix products containing spices and/or herbs as well as optional additional flavoring agents, salt, sugar, and starches.

The palatability improving agent may additionally be an artificial flavoring. The term "artificial" means not derived from natural animal sources. These include the fruit flavors, vegetable flavors, cheese flavors, nut flavors, and the like. Many of these artificial flavors are listed in the Kirk-Othmer Encyclopedia of Chemical Technology, Vol. II, pp 24-28 (1994), the contents of which are incorporated by reference.

Other palatability improving agents include artificial meat, poultry, and fish flavoring agents. These include, for example, such products as artificial beef or vegetarian beef, artificial or vegetarian pork products, including vegetarian ham, vegetarian bacon, vegetarian sausage, artificial poultry (i.e. turkey, chicken, and the like) products, artificial fish products, and the like.

In addition, the palatability improving agent may be derived from yeast. Yeast from the group asomycetous or asporagenous may be utilized. Also included are the yeast like genera which belong to the order, Ustilaginales (in the Basidiomycetes) and the yeast like genera which belong to the family Sporobolomycetes and Sporobolomycetaceae. However, it is preferred that the yeast are commercially available dried yeast, such as a primary dried yeast, i.e. *Saccharomyces cerevisiae,* primary dried torula yeast, i.e. *Torulopsis utilis,* and secondary yeast, i.e. brewer's dried yeast, i.e. *Saccharomyces cerevisiae,* and *Saccharomyces carlsbergensis.*

In addition, the palatability improving agent may be derived from a plant source, i.e. soy meal or cotton seed oil.

The palatability improving agents utilized in the present invention are non-toxic and are food acceptable. They are preferably digestible and do not have any adverse gastrointestinal side effects associated therewith, e.g. excess flatulence or gastrointestinal pains, and the like. Moreover the palatability improving agent is one that does not significantly affect the efficacy of the pharmaceutical active ingredient with which it is associated, i.e., it does not inhibit significantly and more preferably does not inhibit the action of the drug.

Preferred palatability improving agents include hydrolyzed vegetable protein, blends of natural flavoring and spices such as Sirius Stuff ™ and Dog Bone marinade ®, manufactured by Dirigo Corp, vegetarian beef, vegetarian bacon, and roast garlic, manufactured by Geneva Ingredients, Inc., blends of dried skim milk, malted milk, whey, and other products, such as All dairy Blend™, yeast flavoring, especially 100% *Saccharomyces cerevisiae,* such as Brewtech ™ Dried Brewer's Yeast, blends of animal proteins and fat formulated to replace whole egg, such as Eggsact ™, and blends of white and yellow cheese product powders, and cheese rind such as Cheese Plus Cheese™, manufactured by International Ingredients Corp, peanut butter and artificial Chicken, manufactured by Bush Bake Allan Americas, artificial beef manufactured by Pharmachemie at Syracuse, Nebraska or mixtures thereof.

The palatability improving agent is present in the palatable pharmaceutical composition in amounts effective to make the pharmaceutical palatable to the companion animal and if the pharmaceutical has an unacceptable flavor, in amounts effective to mask the off flavor, i.e., palatability improving amounts. It is preferred that the palatability improving agent, except yeast, be present in amounts ranging from about 0.025% to about 99% by weight of the pharmaceutical dosage form. More preferred is the palatability improving agent, except yeast, is present in the amounts ranging from about 0.75% to about 50% and most preferably from about 1% to about 25% by weight of the palatable pharmaceutical composition. With respect to the yeast flavoring, it is preferred that the yeast be present in amounts ranging from about 2% to about 25% by weight of the pharmaceutical compositions, more preferably from about 5% to about 20% by weight of the pharmaceutical composition.

The palatability improving agent is given to the companion animal in association with veterinarian drugs normally given to companion animals. The oral medicaments include such drugs as amebicides, trichomonacides, analgesics, anorexics, antiarthritics, antibacterials, antibiotics, anticoagulants, antidepressants, antihistamines, antineoplastics, antiParkinsonism drugs, antipyretics, anti-spasmodics, anticholinergics, antiviral agents, cardiovascular drugs, contraceptives, diuretics, fertility agents, hematinics, hormones, laxatives, parasympathetic agents, parasympathomometics, psychostimulants, sedatives, sympathomimetics, anti-inflammatory agents, barbiturates, stimulants, tranquilizers, and the like. Examples include carprofen, selegeline, icopezil, methamphetamine, methcyclothiazide, cephalexmin, cephaloglycin, cloxacillin, phenoxyethyl penicillin, erythromycin, pargyline, ephedrine, codeine, methycyclothiazide, metharbital, deserpidine, pentobarbital, isoproterenol, piperazine, estrone, hydrochlorothiazide, ethchlorvynol, chlorazepate, sulfamethizole, phenazopyridine, oxytetracycline, pentaerythritol tetranitrate, diethylstilbestrol, 1-hyoscyamine, ethaverine, pentylenetetrazol, griseofulvin, ampicillin, phendimetrazine, meprobamate, conjugated estrogens, testosterone, pralidoxime, dicloxacillin, isoniacid, methanamine mandelate, phenacetain, aspirin, caffeine, hydrocodone bitartrate, oxacillin, phentermine, bisacodyl NF, phenmetrazine, ephedrine, glyceryl guaiacolate, phenobarbital, theophylline, sulfonamide, phenoxymethyl penicillin, kanamycin, tetracycline, hetacillin, metampicillin, aluminum glycinate, acetaminophen, salicylamide, methyltestosterone, bephenium hydroxynaphthoate, erythrityl tetranitrate, procyclidine, digoxin, cyclizine trimethoprim, sulfamethoxazole, benzyl penicillin, papaverine, hydralazine, allobarbital, acetaminophen, methandrostenolone, dimethindene, xylometazoline, tolazoline, tripenalennamine, reserpine, adiphenine, ethinamate, belladonna, piperacetazine, rifampin, warfarin, promethazine, sulfinpyrazone, phenylbutazone, oxyphenbutazone, carbamazepine, imipramine, furosemide, glycerol trinitrate, isoproterenol, bromisovalum, pentylenetetrazol, isometheptene, oxyphenonium bromide, amantadine, lithium carbonate, butyrophenone, hydroxyzines, chorionic gonadotropin, menotropins, cyanocobalamin, dipyridamole, casanthranol, dioctyl sodium sulfosuccinate, methylphenidate, thyroxine, amphetamine, chlordiazepoxide, diazepam and sulfisoxazole, Cephalexin; Chloramphenicol; Lincomycin; Lincomycin hydrochloride monohydrate; Oxytetracycline; Tetracycline; Tylosin, Salicylazosulfapyridine ("Azulfidine"); Sulfadimethoxine; Trimethorprim-sulfadiazine ("Tribrisssen"), Corticotropin (ACTH); Cortisone acetate; Deoxycorticosterone acetate (DOCA); Dexamethoasone; Hydrocortisone acetate; Phenylbutazone; Prednisolone, Mibolerone; Progesterone; L-Thyroxin (T₄, tetraiodothyronine), Aracoline acetarsol; Arecoline hydrobromide; Bephenium embonate (or hydroxynaphthoate); Bunamidine hydrochloride; Diethylcarbamazine citrate; Dichlorophen; Disophenol; Hexylresorcinol; Mebendazole; Niclosamide; Piperazine salts, Barbituric acid, Phenobarbital sodium, thiopental sodium, Amphetamin, dextroamphetamine, Diphenylhydantoin, Phenobarbital, Acepromazine maleate; Chlorpromazine; Meperidine hydrochloride; Meprobamate, Norpinephrin, epinephrine, isoproternol, ephedrine, atropine, methscopolamine, Chlorpheniramine maleate; Tripelennamine, Amphetamine sulfate; Bethanechol chloride; Cyclophosphamide; Mitotane (o,p' DDD); D-Penicillamine, Mercaptomerin, chlormerodrin, acetazolamide, cyclothiazide, chlorothiazide, Meperidine, Darbazine, Digoxin, quinidine, procainamide, lidocaine, aminophylline, amlodipine, amloipine besylate, and the like.

In general, any improvement in acceptance of a palatable dosage form containing pharmaceutically active ingredients is desirable over pharmaceutical dosage forms that are not formulated to increase palatability. It is preferred that the palatable dosage form have an acceptance rate of about 30% or greater. More preferred is a palatable dosage form with an acceptance rate of about 50% or greater. Even more preferred is a palatable dosage form with an acceptance rate of about 80% or greater. Most preferred is a palatable dosage form with an acceptance rate of about 90% or greater.

The pharmaceutically active agent is present in amounts effective to treat a particular disease or in prophylactically effective amounts. The pharmaceutically effective amount varies with each drug and is determined by the veterinarian prescribing the drug.

The veterinarian will determine the dosage of the present pharmaceutically active agents which will be most suitable. The amount will depend upon several factors. For example, it will vary with the form of administration and the particular compound chosen, and furthermore, it will vary with the animal under treatment, the age of the animal, the weight of the animal and the type of malady being treated. However, the effective amount of drug to be delivered would be no different if palatability improving agent were not present.

The palatable pharmaceutical composition may be orally administered, for example, with an inert diluent or with an assimilable edible carrier, or it may be enclosed in hard or soft shell gelatin capsules, or it may be compressed into tablets, or in the form of troches, or it may be incorporated directly into the food of the diet. For oral therapeutic administration, the active compound, and the flavoring agent may be incorporated with excipients and used in the form of ingestible tablets, troches, capsules and wafers, or alternatively, can be administered in liquid form.

The palatability improving agent can be added as a coating to the dosage form, such as a tablet or pill containing the pharmaceutically active agent. Alternatively, the palatability improving agent can be sprayed onto the surface of a table or pill containing the pharmaceutical agent. The palatable pharmaceutical composition of the invention can also be a chewable tablet.

Preferably, the palatability improving agent and the pharmaceutically active agent are compressed into tablets. More preferably, such tablets exhibit a hardness range of from about 5 to about 25 kP. Preferably, such tablets have less than about 7.5% free or adsorbed water content as measured by loss on drying. The water does not include water of hydration present in the active pharmaceutical ingredient or any of the excipients, including the flavor enhancing ingredients. More preferably, the water content is less than about 5%. At this water activity, the pharmaceutical compositions of the present invention are relatively free from mold or bacteria growth contamination. However, the pharmaceutical composition may also contain an anti-mycotic and/or anti-bacterial agent.

Preferably, the palatability improving agent increases the shelf life of the pharmaceutically active agent.

Furthermore, the palatability improving agent enhances compliance with a therapeutic program for companion animals.

The tablets, troches, pills, capsules and the like may also contain the following: a binder such as sodium starch glycosate, gum tragacanth, acacia, polyvinylpyrrolidone, corn starch or gelatin; excipients such as dicalcium phosphate, and microcrystalline cellulose; a disintegrating agent such as corn starch, potato starch, alginic acid, and the like; a lubricant such as magnesium stearate, stearic acid, polyethylene glycol, talc or silica. When the dosage unit form is a capsule, it may contain, in addition to materials of the above type, a liquid carrier.

Coatings or other components may be present so as to modify the physical form of the pharmaceutical composition. For instance, tablets, pills, or capsules may be coated with shellac, sugar or both, sweetening agent, methyl and propylparabens as preservatives, coloring agents, a dye and other ingredients such as cherry. The palatable pharmaceutical composition is preferably prepared in unit dosage form. In such form, the preparation is subdivided into unit doses containing appropriate quantities of the pharmaceutically active agent in association with the palatability improving agent. The unit dosage form can be in packaged preparation, such as packaged tablets, capsules, pills, lozenges, troche and the like. The preferred solid unit dosage form is a hard, compressed tablet. Of course, any material used in preparing any dosage unit form should be pharmaceutically pure and substantially non-toxic in the amounts employed.

As used herein, "pharmaceutically acceptable carrier" includes any and all solvents and dispersion media for pharmaceutically active substances are well known in the art. Except insofar as any conventional media or agent is incompatible with the active ingredient, its use in the therapeutic compositions of the present invention is contemplated. More than one active ingredient can also be incorporated into the palatable pharmaceutical compositions.

The preparation of the palatable pharmaceutical compositions of the present invention can be accomplished by utilizing any one of a wide variety of different known methods. One such method is by wet granulation in which the components, e.g., pharmaceutically active agent, the palatability improving agent, and excipients are mixed with a wet granulating solvent, such as an aqueous or a non-aqueous solvent or solvent mixtures, e.g., as water or alcohol, in a mixing apparatus. The mixture is dried using techniques known in the art and then the dried blend is generally processed further by sizing the granulation through a mill to reduce the size of the particles. Lubricant and any additional excipients are then added and blended to provide a uniform homogeneous mixture. In another variation, the blend is simultaneously granulated in the granulating vehicle and dried using a fluid bed granulation process. The resulting granules are milled and then blended with a lubricating agent.

The palatable pharmaceutical compositions of the present invention may be prepared by dry formulation in which the pharmaceutically active agent, the palatability improving agent and the carrier material are thoroughly intermixed. Excipients, binding agents, lubricants, disintegrating agents, and colorants, if necessary are homogeneously mixed. Examples of suitable excipients are lactose and sodium starch glycolate.

The resulting blend is then made into solid dosage form. If the final dosage form is a tablet or chewable tablet, the composition is transferred to a tablet press and compressed into a tablet at an appropriate compression pressure to achieve, preferably, a harness in the range of from about 5 to about 25 kP, at compression pressures of about 1600 to about 2000 pounds/square inch. The product thus obtained has the desired hardness, and low level of friability found in tablets.

Alternatively, the blend may be encapsulated by a gelatin shell to form a capsule utilizing techniques known to one of ordinary skill in the art. Similarly, pills and troches are prepared using conventional techniques known to the skilled artisan.

Alternatively, the palatable pharmaceutical compositions of the present invention may be formed into shapes, textures, and mimic structures so as to simulate foods, such as biscuits, cheeses, meat scraps and the like.

The present inventors have found that when the palatability improving agents of the present invention are added to pharmaceutically active agents, the companion animals were not only attracted thereto, but also freely ingested and swallowed the pharmaceutical compositions containing the palatability improving agents. These are described in the following examples.

Moreover, the present inventors have found that the addition of the palatability improving agents in palatable enhancing effective amounts made the pharmaceutical compositions more stable and increased shelf-life when the palatability improving agent of the present invention was present. Unless indicated to the contrary, the percentages are by weight of the pharmaceutically composition.

The term "effective" amount of a drug is meant a non-toxic but sufficient amount of compound to provide the desired therapeutic or prophylactic effect.

"Carriers" or "vehicles" as used herein refer to carrier material suitable for solid oral drug administration and include any such materials known in the art, e.g., diluent, binders, granulating agents, disintegrates, lubricating agents, colorants and the like.

The flavoring agents used in the Examples are as follows:
Vegetarian beef flavor, Geneva Ingredients, Inc, Waunakee, WI, is a mixture of maltodextrin, autolyzed yeast extract, natural flavors, partially hydrogenated vegetable oil (soybean and/or cottonseed), onion powder, and silicon dioxide. Vegetarian bacon flavor, Geneva Ingredients, Inc., Waunakee, WI, is a mixture of maltodextrin, natural flavors, peanut oil, natural smoke flavor, and silicon dioxide.
Roast garlic flavor, Geneva Ingredients, Inc., Waunakee, WI, is a mixture of salt, maltodextrin, autolyzed yeast extract, natural flavors, partially hydrogenated vegetable oil (cottonseed or soybean) and silicon dioxide.
Artificial powdered beef flavor, Pharma Chemie, Lincoln, NE, is a mixture of hydrolyzed vegetable protein, natural flavor, and hydrogenated vegetable oils.
Brewtech® Dried Brewers Yeast, International Ingredient Corporation, St. Louis, MO, is 100% dried Saccharomyces cerevisiae from the brewing industry that is distilled to remove the alcohol, naturally debittered, and roller dried.
Eggsact™, Dried egg replacer, International Ingredient Corporation, St. Louis, MO, is a special blend of animal proteins and fat formulated to replace or extend whole eggs.
Cheese Plus Cheese product, International Ingredient Corporation, St. Louis, MO, is a blend of white and yellow cheese product powders, and cheese rind.
Sugar foods by-product, International Ingredient Corporation, St. Louis, MO, is produced from the by-products of dry packaged drink mixes, dried gelatin mixes, hard candy, and similar specialty food products that have a high sugar content, and citric acid.
Trusil N/A Peanut flavor, Bush Boake Allen Americas, Chicago, IL, is a trade-secret mixture of flavor items on the FEMA/GRAS list.
Artificial chicken flavor, Bush Boake Allen Americas, Chicago, IL, is a trade-secret mixture of flavor items on the FEMA/GRAS list.
Sirius Stuff™, Dirigo Corporation, Boston, MA, is a blend of yeast, garlic, salt, herbs, kelp and fermented soy.

The following non-limiting Examples further illustrate the invention.

### EXAMPLE 1

Palatable formulations of carprofen were made as follows:

A carprofen containing granulation was prepared by a conventional aqueous high shear wet granulation process with 27.50% carprofen, 4.93% sodium starch glycolate, 4.93% pregelatinized starch, and 59.73% lactose followed by fluid bed drying. The following components were added to the dried milled granulation: 0.5% silicon dioxide, 1.6% talc, and 0.8% magnesium stearate and the components were mixed. To the carprofen blend described hereinabove, were added a flavoring component listed in Table 1. The resulting blend was compressed into tablets of hardness > 10 Kp using 0.504" X 0.252" tooling.

**Table 1:**

| Compositions by weight (in milligrams) of typical palatable formulations of carprofen. | | | | | | |
|---|---|---|---|---|---|---|
| | BA | BB | BC | BD | BE | BF |
| Carprofen blend | 366.2 | 361.8 | 361.8 | 361.8 | 361.8 | 361.8 |
| Cheese Plus | 29.7 | | | | | |
| Sirius Stuff | | 27.2 | | | | |
| Artificial Chicken | | | 19.0 | | | |
| BrewTech Yeast | | | | 63.8 | | |
| Vegetarian Beef | | | | | 7.4 | |
| Vegetarian Bacon | | | | | | 7.4 |
| Tablet hardness (Kp) | 10.7-13.5 | 12.1-13.4 | 10.0 - 12.9 | 10.8 - 14.1 | 11.0 - 14.3 | 10.5 - 13.4 |
| Total Tablet Weight (mg) | 395.9 | 389.0 | 380.8 | 425.6 | 369.2 | 369.2 |

### EXAMPLE 2

Flavored placebo tablets for palatability testing were made by blending the ingredients given in Table 2A and Table 2B and compressing the blend into 300 mg total weight tablets using 0.458" x 0.229" tooling.

**Table 2A:**

| Compositions of flavored small placebo tablets | | | | | |
|---|---|---|---|---|---|
| | 42A | 42B | 42C | 42D | 42E |
| Microcrystalline cellulose | 25.0% | 25.0% | 25.0% | 25.0% | 25.0% |
| Lactose | 71.5% | 54.0% | 73.4% | 67.0% | 72.0% |
| Artificial Chicken | 2.5% | | | | |
| Artificial Powdered Beef | | 20.0% | | | |
| Artificial Peanut | | | 0.6% | | |
| Sirius Stuff | | | | 7.0% | |
| Roast Garlic | | | | | 2.0% |
| Magnesium stearate | 1.0% | 1.0% | 1.0% | 1.0% | 1.0% |

**Table 2B:**

| Compositions of flavored small placebo tablets | | | | | | | | | |
|---|---|---|---|---|---|---|---|---|---|
| | 47A | 47B | 47C | 47D | 47E | 47F | 47G | 47H | 471 |
| Microcrystalline cellulose | 25.0 | 25.0 | 25.0 | 25.0 | 25.0 | 25.0 | 25.0 | 25.0 | 25.0 |
| Lactose | 73.25 | 59.0 | 59.0 | 59.0 | 59.0 | 59.0 | 67.0 | 73.0 | 73.0 |
| Trusil Ham Flavor | 0.75 | | | | | | | | |
| Sugar Food By-Products | | 15.0 | | | | | | | |
| Eggsact Dried Egg Replacer | | | 15.0 | | | | | | |
| Cheese Plus Cheese | | | | 15.0 | | | | | |
| All Dairy Blend | | | | | 15.0 | | | | |
| BrewTech Dried Yeast | | | | | | 15.0 | | | |
| Dog bone | | | | | | | 7.0 | | |
| Marinade | | | | | | | | | |
| Veg. Beef Type | | | | | | | | 1.0 | |
| Veg. Bacon Type | | | | | | | | | 1.0 |
| Magnesium stearate | 1.0 | 1.0 | 1.0 | 1.0 | 1.0 | 1.0 | 1.0 | 1.0 | 1.0 |

### EXAMPLE 3

Flavored placebo tablets for palatability testing were made by blending the ingredients given in Table 3A and Table 3B and compressing the blend into 600 mg total weight tablets using 0.635" x 0.3175" tooling.

**Table 3A:**

| Compositions of flavored large placebo tablets | | | | | | | | |
|---|---|---|---|---|---|---|---|---|
| | A | B | C | D | E | F | F | H |
| Microcrystalline cellulose | 25.0 | 25.0 | 25.0 | 25.0 | 25.0 | 25.0 | 25.0 | 25.0 |
| Lactose | 59.0 | 66.5 | 72.0 | 70.0 | 71.5 | 69.0 | 67.0 | 70.5 |
| Eggsact Dried Egg Replacer | 15.0 | 7.5 | | | | | | |
| Roast Garlic | | | 2.0 | 4.0 | | | | |
| Artificial Chicken | | | | | 2.5 | 5.0 | | |
| Sirius Stuff | | | | | | | 7.0 | 3.5 |
| Magnesium stearate | 1.0 | 1.0 | 1.0 | 1.0 | 1.0 | 1.0 | 1.0 | 1.0 |

**Table 3B:**

| Composition of flavored large placebo tablets | | | | | | | | | | |
|---|---|---|---|---|---|---|---|---|---|---|
| | A | B | C | D | E | F | G | H | I | J |
| Microcrystalline cellulose | 25.0 | 25.0 | 25.0 | 25.0 | 25.0 | 25.0 | 25.0 | 25.0 | 25.0 | 25.0 |
| Lactose | 59.0 | 66.5 | 73.0 | 72.0 | 59.0 | 66.5 | 73.0 | 72.0 | 67.0 | 64.0 |
| BrewTech Yeast | 15.0 | 7.5 | | | | | | | | |
| Veg. Bacon | | | 1.0 | 2.0 | | | | | | |
| Cheese Plus Cheese | | | | | 15.0 | 7.5 | | | | |
| Veg. Beef | | | | | | | 1.0 | 2.0 | | |
| Dog Bone Marinade | | | | | | | | | 7.0 | 10.0 |
| Magnesium stearate | 1.0 | 1.0 | 1.0 | 1.0 | 1.0 | 1.0 | 1.0 | 1.0 | 1.0 | 1.0 |

### EXAMPLE 4

### Canine Acceptance Test

A canine acceptability test was conducted in random source dogs using flavored tablets. The purpose of this study was to screen several flavors to identify if any of the test-flavors were unacceptable to dogs. Therefore, an optimal presentation was used in which dogs were offered a single tablet by hand in a test period and were allowed to accept or reject the tablet.

Dogs participated in three test periods on day 0 and day 1, and in two test periods on day 2, for a total of 8 test tablet offerings. Each flavor test within a day was separated by a minimum 3 hour "rest" phase before the next test was conducted. Each dog was offered each flavor only one time during the test. For each flavor, 5 dogs were tested at each test period. Within these constraints, the flavors were randomly assigned to individual dogs.

Dogs were weighed within a 24 hour period prior to the start of the study. An animal handler, known well to the dog, offered tablets to each dog. For each test tablet offering, records were kept of the following: dog ID, the tablet flavor (by flavor code number), whether or not the animal accepted the tablet, whether the animal consumed or rejected the tablet, and the lapsed time from offering the tablet (start stopwatch) to the time of consumption or rejection (i.e., if the dog spits the tablet out) (stop stopwatch). If the animal did not take the tablet in its mouth within 2 minutes of the offer, the tablet was withdrawn and the dog was scored as NOT having accepted the tablet. If the animal did not consume the tablet within 3 minutes of the offer, the dog was scored as NOT consumed the tablet.

If the dog was known not to accept treats from human beings, but did readily consume treats from the feed bowl, the tablets were presented in the empty feed bowl and timing was begun from the time of tablet placement in the bowl. As much as possible, tablets were offered over the resting pad to prevent the tablet from falling through the floor grating. If the tablet was dropped and landed on the pad, the dog was allowed time to pick it up. If the dog accidentally dropped the tablet through the cage floor, the tablet was offered again as quickly as possible after retrieval. The stopwatch was stopped if a tablet fell through the grating and restarted when the tablet was re-offered. Only one drop per dog was permitted per individual flavor test. If the animal dropped the tablet a second time, the stopwatch was stopped and the time recorded, the tablet discarded and a note of the behavior made in the data form.

Animals were observed daily throughout the study for overall health. The dogs were fed *ad libitum* overnight, with fresh feed provided late in the afternoon and feed removed from the pens at least 2 hours before the morning test period of each study day. For this reason, dogs were acclimated to a similar feeding schedule for at least 5 days prior to the start of the study.

Placebo tablet formulations were made as described in Example 2 and canine acceptance tests were carried out as described hereinabove. The results of the study are given in Table 4A. The correspondence of the flavor codes and the formulations/flavors is given in the table 4B.

**Table 4A**

| Flavor Code | Formulation | Tablet Wt. | % Flavor | Flavor |
|---|---|---|---|---|
| A | 42A | 300 | 2.5 | Artificial Chicken |
| B | 42B | 300 | 20.0 | Artificial Powder Beef |
| C | 42C | 300 | 0.6 | Artificial Peanut |
| D | 42D | 300 | 7.0 | Sirius Stuff |
| E | 42E | 300 | 2.0 | Roast Garlic |
| F | 47A | 300 | 0.75 | Trusil Ham Flavor |
| G | 47B | 300 | 15 | Sugar Food By-Products |
| H | 47C | 300 | 15 | Eggsact Dried Egg Replacer |

**Table 4B:**

| Results of canine acceptance testing | | | | | | | | |
|---|---|---|---|---|---|---|---|---|
| Flavor Code | A | B | C | D | E | F | G | H |
| #dogs tested | 40 | 40 | 40 | 40 | 40 | 40 | 40 | 40 |
| Accept | 38 | 36 | 39 | 40 | 38 | 39 | 39 | 39 |
| Consume | 33 | 29 | 30 | 34 | 35 | 33 | 33 | 38+ |
| Not consumed | 7 | 11 | 10 | 6 | 5 | 7 | 7 | 1+ |
| Total time (sec) | 36 | 62 | 55 | 44 | 34 | 49 | 47 | 22 |
| Consume time (sec) | 21 | 28 | 24 | 28 | 14 | 25 | 27 | 18 |
| SCORE | 4 | 8 | 7 | 3 | 2 | 5 | 5 | 1 |
| Dogs/#1 flavor** | 11 | 3 | 3 | 7 | 9 | 1 | 3 | 14 |
| Dogs/#8 flavor | 7 | 12 | 10 | 4 | 5 | 7 | 7 | 2 |

| | | | | | | | | |
|---|---|---|---|---|---|---|---|---|
| ** This row represents the number of dogs with the BEST response for a given flavor. | | | | | | | | |

The next row represents the WORST response to a given flavor.

### EXAMPLE 5

Placebo tablet formulations were made as described in Example 2 or Example 3 and canine acceptance tests were carried out as described in Example 4. The correspondence of the flavor codes and the formulations/flavors is given in Table 5A. The results of the study are given in Table 5B.

**Table 5A**

| Flavor Code | Formulation | Tablet Wt. | % Flavor | Flavor |
|---|---|---|---|---|
| J | 47D | 300 | 15 | Cheese Plus Cheese |
| K | 47C | 300 | 15 | Eggsact Dried Egg Replacer |
| L | 47E | 300 | 15 | All Dairy Blend |
| M | 47F | 300 | 15 | BrewTech Dried Brewers Yeast |
| N | 47G | 300 | 7 | Dog bone Marinade |
| P | 42E | 300 | 2 | Roast Garlic |
| Q | 47H | 300 | 1 | Vegetarian Beef Type Flavor |
| R | 471 | 300 | 1 | Vegetarian Bacon Type Flavor |

**Table 5B:**

| Results of canine acceptance testing | | | | | | | | |
|---|---|---|---|---|---|---|---|---|
| Flavor Code | J | K | L | M | N | P | Q | R |
| #dogs tested | 40 | 40 | 40 | 40 | 40 | 40 | 40 | 40 |
| Accept | 40 | 39 | 40 | 40 | 39 | 39 | 40 | 40 |
| Consume | 39 | 38+ | 40 | 40 | 37 | 37 | 39 | 39 |
| Not consumed | 1 | 1+ | 0 | 0 | 3 | 3 | 1 | 1 |
| Total time (sec) | 14 | 15 | 13 | 11 | 17 | 15 | 12 | 13 |
| Consume time (sec) | 10 | 11 | 13 | 11 | 8 | 9 | 12 | 11 |
| SCORE | 2 | 8 | 5 | 1 | 7 | 6 | 4 | 2 |
| Dogs/#1 flavor** | 8 | 16 | 3 | 9 | 10 | 14 | 7 | 7 |
| Dogs/#8 flavor | 6 | 4 | 5 | 7 | 4 | 5 | 6 | 7 |

| | | | | | | | | |
|---|---|---|---|---|---|---|---|---|
| ** This row represents the number of dogs with a BEST response for a given flavor. The next row represents the least favorable response to a given flavor. | | | | | | | | |

### EXAMPLE 6

Placebo tablet formulations were made as described in Example 2 or Example 3 and canine acceptance tests were carried out as described in Example 4. The correspondence of the flavor codes and the formulation/flavors is given in Table 6A.The results of the study are given in Table 6B.

**Table 6A**

| Flavor Code | Formulation | Tablet Wt. | % Flavor | Flavor |
|---|---|---|---|---|
| S | 70E | 600 | 2.5 | Artificial Chicken |
| T | 70H | 600 | 3.5 | Sirius Stuff |
| U | 70B | 600 | 7.5 | Eggsact Dried Egg Replacer |
| V | 70D | 600 | 4.0 | Roast Garlic |
| W | 70A | 600 | 15.0 | Eggsact Dried Egg Replacer |
| X | 70C | 600 | 2.0 | Roast Garlic |
| Y | 70F | 600 | 5.0 | Artificial Chicken |
| Z | 70G | 600 | 7.0 | Sirius Stuff |

**Table 6B:**

| Results of canine acceptance testing | | | | | | | | |
|---|---|---|---|---|---|---|---|---|
| Flavor Code | S | T | U | V | W | X | Y | Z |
| #dogs tested | 40 | 40 | 40 | 40 | 40 | 40 | 40 | 40 |
| Accept | 40 | 40 | 40 | 40 | 40 | 40 | 40 | 40 |
| Consume | 40 | 40 | 40 | 39 | 40 | 39 | 40 | 40 |
| Not consumed | 0 | 0 | 0 | 0 | 1 | 0 | 1 | 0 |
| Total time (sec) | 12.6 | 11.5 | 11.3 | 13 | 8.5 | 15.4 | 13.1 | 9.8 |
| SCORE | 5 | 4 | 3 | 7 | 1 | 8 | 6 | 2 |

### EXAMPLE 7

Placebo tablet formulations were made as described in Example 2 or Example 3 and canine acceptance tests were carried out as described in Example 4. The correspondence of the flavor codes and the formulations/flavors is given in Table 7A. The results of the study are given in Table 7B.

**Table 7A**

| Flavor Code | Formulation | Tablet Wt. | % Flavor | Flavor |
|---|---|---|---|---|
| AA | 72E | 600 | 15 | Cheese Plus Cheese |
| AB | 72H | 600 | 2 | Vegetarian Beef Type Flavor |
| AC | 72B | 600 | 7.5 | BrewTech Dried Brewers Yeast |
| AD | 72G | 600 | 1 | Vegetarian Beef Type Flavor |
| AE | 72A | 600 | 15 | BrewTech Dried Brewers Yeast |
| AF | 72F | 600 | 7.5 | Cheese Plus Cheese |
| AG | 72D | 600 | 2 | Vegetarian Bacon Type Flavor |
| AH | 72C | 600 | 1 | Vegetarian Bacon Type Flavor |

| Table 7B: Results of canine acceptance testing | | | | | | | | |
|---|---|---|---|---|---|---|---|---|
| Flavor Code | AA | AB | AC | AD | AE | AF | AG | AH |
| #dogs tested | 40 | 40 | 40 | 40 | 40 | 40 | 40 | 40 |
| Accept | 40 | 40 | 40 | 40 | 40 | 40 | 40 | 39 |
| Consume | 39 | 39 | 39 | 39 | 40 | 40 | 39 | 38 |
| Not consumed | 1 | 1 | 1 | 1 | | | 1 | 2 |
| Total time (sec) | 7.45 | 12.18 | 7.82 | 11.5 | 8.5 | 7.65 | 13.3 | 15.3 |
| SCORE | 2 | 6 | 4 | 5 | 3 | 1 | 7 | 8 |

### EXAMPLE 8

Placebo tablet formulations were made as described in Example 2 or Example 3 and canine acceptance tests were carried out as described in Example 4. The results obtained are of a similar nature as given in Example 4 and Example 5. A summary of the results of canine acceptability tests of small and large placebo tablets and the corresponding flavors and their concentration in the tablets (ratio of palatability improving agent to the total tablet weight) are given in Tables 8A and Table 8B below.

**Table 8A:**

| Summary of canine acceptance tests (small placebo tablets) | | | | | | | |
|---|---|---|---|---|---|---|---|
| Palatability improving agent | Concentration (%) | # Dogs Tested | Tablets Tasted | Tablets Consumed | Not Consumed | Total Time (sec) | Acceptance Rank |
| Artificial Chicken | 2.5% | 40 | 38 | 33 | 7 | 36 | 5 |
| Sirius Stuff | 7% | 40 | 40 | 34 | 6 | 44 | 2 |
| Cheese + Cheese | 15% | 40 | 40 | 39 | 1 | 14 | 5 |
| BrewTech Yeast | 15% | 40 | 40 | 40 | 0 | 11 | 1 |
| Vegetarian Beef | 1% | 40 | 40 | 39 | 1 | 12 | 3 |
| Vegetarian Bacon | 1% | 40 | 40 | 39 | 1 | 13 | 4 |

**Table 8B:**

| Summary of canine acceptance tests (large placebo tablets) | | | | | | | |
|---|---|---|---|---|---|---|---|
| Palatability improving agent | Concentration (%) | # Dogs Tested | Tablets Tasted | Tablets Consumed | Not Consumed | Total Time (sec) | Acceptance Rank |
| Artificial Chicken | 5% | 40 | 40 | 40 | 0 | 13 | 6 |
| Sirius Stuff | 7% | 40 | 40 | 40 | 0 | 10 | 2 |
| Vegetarian Beef | 2% | 40 | 40 | 39 | 1 | 12 | 6 |
| BrewTech Yeast | 15% | 40 | 40 | 40 | 0 | 8 | 3 |
| Cheese + Cheese | 7.5% | 40 | 40 | 40 | 0 | 8 | 1 |
| Vegetarian Bacon | 2% | 40 | 40 | 39 | 1 | 13 | 7 |

### EXAMPLE 9

A canine Flavor Preference Test was conducted to evaluate preferences random source dogs may have for three different flavors when incorporated into pillable tablets containing carprofen. Since the purpose of this study was to determine if any of the flavors were substantially more or less preferred than another flavor, paired presentations were necessary. A dog may refuse to consume either or both tablets in a paired presentation, but a dog must also have the opportunity to consume both tablets to permit assessment of comparably good partners in a flavor pair.

A total of three flavors were tested in the study with each flavor being incorporated in approximately 400 mg total tablet weight each containing 100 mg carprofen. Dogs participated in two test periods per day on days 0 through day 9 for a total of 18 paired test offerings per dog. Each flavor test within a day was separated by a minimum 9 hour "rest" phase before the next test was conducted. Each dog was offered each flavor 12 times within the study (6 times with each of the other two flavors). For each flavor pair, 10 dogs were tested at each test period. Within these constraints, the three different flavor pairs were randomly assigned within the 18 dosing schedule for each dog. The study was conducted as a blinded evaluation of the rank order of selection and consumption preference for the three different tablets.

Dogs were weighed within 24 hours prior to the start of the study. All dogs weighed at least 12kg. A pair of tablets (200 mg carprofen) were offered to each dog in a test period in a two-position rack with 2 disposable food bowls. A single tablet was placed in each bowl and a corresponding position code was clearly visible to the recording technician. A hinged lid was placed over the food bowls to prevent access to the tablets until the test was started.

Prior to the initiation of the preference testing, dogs were permitted sufficient time to become accustomed to the testing rack and the test procedures by offering commercially available dog treats in the test bowls. Selection and feeding behavior, which may result in unscored test outcomes, were used as criteria to reject dogs from the study. In addition, dogs that were behaviorally or physically unsuitable were excluded from the study.

Palatability preference testing was conducted twice daily on each of 9 consecutive days. Dogs did not receive food for at least 1 hour prior to the start of each test. Tests were. done in the morning and afternoon when there were no other activities being conducted in the room that might distract the dogs.

To begin a test, the covered 2 bowl test rack was placed in the dog's pen. Each bowl contained a different tablet. The location of each tablet within the test rack on each day for each dog was randomly pre-determined, with the restriction that each tablet was placed in each position an equal number of times in the course of the study. Upon the start of a test (time=0), the lid was raised to allow dogs access to the tablets. An observer determined the order in which each tablet was selected by the dog. Selection was defined as the intentional removal of the tablet from the bowl by the dog's mouth. Tablets were also scored on the basis of the order in which they were consumed. A tablet was not ranked and scored as consumed until it was completely swallowed. Tablets were assigned rank scores of 1 or 2 corresponding to the order in which they were selected or consumed. Time allowed for each test was three minutes. If a tablet was not selected or consumed by that time, it was given a score of 3. If a tablet was dropped into an inaccessible location (by dog) after selection, the tablet was not given a consumption score.

Dogs were fed *ad libitum,* with fresh feed provided in the morning and afternoon following each test. Feed was removed from the pens at least one hour before each test period. For this reason, dogs were acclimated to a similar feeding schedule for at least 5 days prior to the start of the study.

Tablets were made as described in Example 1 and tested in the Canine Tablet Preference Study as described hereinabove. Overall the consumption of tablets in the preference testing was acceptable, with 89.5% and 93.3% of the tablets consumed in Studies 1 and 2, respectively. Based on the level of consumption, all of the flavors tested were accepted by most (24) dogs. The overall results are shown in Table 9.

**Table 9:**

| Tablet consumption by dogs in two carprofen tablet preference studies | | | | | | |
|---|---|---|---|---|---|---|
| # Dogs (%) | Total Tablet Summary | | | # Rejections by Flavor | | |
| | % Consumed | % Rejected | % Total Dropped | Cheese + Cheese 7.5% | Sirius Stuff 7% | Artificial Chicken 5% |
| 24(80) | 99.4 | 0 | 0.6 | | | |
| 1 (3) | 52.8 | 2.8 | 44.4 | 1 | 0 | 0 |
| 1 (3) | 88.9 | 11.1 | 0 | 1 | 0 3 | |
| 1 (3) | 63.9 | 22.2 | 13.9 | 1 | 3 | 4 |
| 1 (3) | 61.1 | 36.1 | 2.8 | 0 | 8 | 5 |
| 1 (3) | 36.1 | 47.2 | 2.8 | 4 | 7 | 6 |
| 1 (3) | 0 | 55.6 | 44.4 | 8 | 7 5 | |
| TOTAL 30 | 89.5 | 5.9 | 4.6 | 15 | 25 | 23 |
| | | | | | | |

| | | | | BrewTech Yeast 15% | Vegetarian Beef 2% | Vegetarian Bacon 2% |
|---|---|---|---|---|---|---|
| 24 (80) | 99.3 | 0 | 0.7 | | | |
| 2 (7) | 93.1 | 2.8 | 4.2 | 0 | 1 | 1 |
| 1 (3) | 75.0 | 16.7 | 8.3 | 1 | 3 2 | |
| 1 (3) | 66.7 | 19.4 | 13.9 | 0 | 2 | 5 |
| 1 (3) | 52.8 | 36.1 | 11.1 | 2 | 4 | 7 |
| 1 (3) | 36.1 | 41.7 | 22.2 | 6 | 4 5 | |
| TOTAL 30 | 93.3 | 4.0 | 2.7 | 9 | 14 | 20 |

### EXAMPLE 10

Tablets were made as described in Example 1 and tested in the Canine Tablet Preference Study as described in Example 9. The preferences for flavors are further delineated in Table 10. Based on consumption, all six flavors were well accepted by the dogs tested with consumption ranging from 92.7% to 97.5% of tablets that were not dropped out of the dogs' reach.

Cheese + Cheese and BrewTech Yeast were the most consumed in their respective studies. Flavors more frequently rejected were Sirius Stuff and Artificial Chicken in the first study and Vegetarian Bacon in the second study. However, Sirius Stuff was chosen FIRST as least as often as Cheese + Cheese in the first study. In the second study, Vegetarian Bacon flavored tablets were clearly less favored as BrewTech Yeast was highly favored by dogs based on consumption, rejection, and consumption order.

**Table 10:**

| Responses to flavor choices by 30 dogs in two caprofen tablet preference studies. | | | | | | |
|---|---|---|---|---|---|---|
| Flavor | %Dogs with 100% Consumed | Consumable Summary | Tablet | % Dropped of Total Offered | Consumption Order | |
| | | % Consumed | % Rejected | | % Firsts | % Seconds |
| Cheese + Cheese | 83.3 | 95.7 | 4.3 | 3.9 | 49.6 | 46.1 |
| Sirius Stuff | 83.3 | 92.7 | 7.3 | 4.7 | 50.7 | 41.9 |
| Artificial Chicken | 80.0 | 93.2 | 6.8 | 5.3 | 45.0 | 48.2 |
| BrewTech Yeast | 90.0 | 97.5 | 2.5 | .8 | 58. | 39.0 |
| Vegetarian Beef | 83.3 | 96.0 | 4.0 | 2.5 | 49.0 | 45.0 |
| Vegetarian Bacon | 83.3 | 94.2 | 5.8 | 4.7 | 38.0 | 52.0 |

### EXAMPLE 11

Tablets were made as described in Example 1 and tested in the Canine Tablet Preference Study as described in Example 9. A comparison of tablet rejections within flavor pairs (when a flavor is rejected in association with presentation of another flavor) is summarized in Table 11. In both studies, the two less liked flavors were equally rejected when offered with the more preferred flavors (Cheese + Cheese and BrewTech Yeast). Rejections of the winning flavors were not different between the two flavor pair presentations. Vegetarian Bacon was more frequently rejected when offered with Vegetarian Beef than when offered with BrewTech Yeast.

**Table 11:**

| Summary of carprofen tablet flavor rejections by paired flavors in two preference studies | | | |
|---|---|---|---|
| Paired Flavor | Rejected Flavor | | |
| | Cheese + Cheese | Sirius Stuff | Artificial Chicken |
| Cheese + Cheese | | 10 | 10 |
| Sirius Stuff | 2 | | 8 |
| Artificial Chicken | 4 | 8 | |

| | BrewTech Yeast | Vegetarian Beef | Vegetarian Bacon |
|---|---|---|---|
| BrewTech Yeast | | 8 | 8 |
| Vegetarian Beef | 5 | | 12 |
| Vegetarian Bacon | 4 | 6 | |

### EXAMPLE 12

Flavored pillable placebo tablets for palatability testing in cats were manufactured by a conventional direct compression process in which all the ingredients listed in Table 13 with the exception of magnesium stearate were blended for 20 minutes and then passed through a 40 mesh screen. The powders were blended again for an additional 20 minutes followed by the addition of magnesium stearate and a final blending for 3 minutes. The blends were compressed to a total tablet weight of 300 mg on a single station Type "F" Manesty Tablet Press using 11/32" Standard Round Concave (SRC) tooling.

**Table 13:**

| Typical compositions of flavored placebo tablets for cat palatability studies | | | | | | |
|---|---|---|---|---|---|---|
| Component | AA/AG | AB | AC | AD | AE | AF |
| Lactose | 222.0 | 201.0 | 207.0 | 177.0 | 216.0 | 216.0 |
| Microcrystalline cellulose | 75.0 | 75.0 | 75.0 | 75.0 | 75.0 | 75.0 |
| Sirius Stuff | | 21.0 | | | | |
| Artificial Chicken | | | 15.0 | | | |
| Brewtech Dried Brewer's Yeast | | | | 45.0 | | |
| Vegetarian Beef | | | | | 6.0 | |
| Vegetarian Bacon | | | | | | 6.0 |
| Magnesium stearate | 3.0 | 3.0 | 3.0 | 3.0 | 3.0 | 3.0 |
| Total tablet weight | 300.0 | 300.0 | 300.0 | 300.0 | 300.0 | 300.0 |

### EXAMPLE 13

Palatability and acceptability tests of flavored pillable tablets were conducted in cats as follows.

### PRECONDITIONING

All cats were preconditioned to the study's feed schedule for 7 days prior to testing. Additionally, in order to assess each cats willingness to consume treats, each cat was offered two treats (Pounce Tartar Control, Crunchy Tuna Flavor and Whisker Lickin's Tuna Flavor (crunchy)) on 3 days prior to study start. The treats were offered simultaneously in a metal feed bowl inside the cat's cage, visible and accessible to the cat. Approximately five minutes after the treats were offered, the bowls were removed and the consumption recorded (Yes/No).

### STUDY DESIGN AND PROCEDURE

Five tablet flavors, a negative control (non-flavored placebo tablet), and a positive control (placebo tablet + 2 drops tuna oil) were used in this study. Approximately 16-24 hours prior to tablet offering tuna oil was drawn from a freshly opened can of Star-Kist Tuna in Light Oil and applied (via Pasteur pipette) to each placebo tablet that was to be used as a positive control. Each positive control tablet was left overnight to soak in any oil that may have run-off during initial application.

Each cat participated in two test periods per day for 7 days, for a total of 14 tablet offerings per cat. Each cat was tested on each flavor and each control two times during the study. The order in which the flavors were offered was random for each cat. Test periods within the same day were separated by a minimum of 3 hr.

Tablets were offered to each cat by the same individual at each offering. The tablets were placed in a metal feed bowl inside the cat's cage, visible and accessible to the cat. Timing (by stopwatch) began when the tablet was placed in the bowl. Tablets were offered over a solid surface to help prevent a tablet from falling out of a cat's reach. When a tablet was dropped within a cat's reach, no intervention was made and the stopwatch continued to run. When a cat dropped a tablet out of reach, the tablet was placed in the feed bowl again as quickly as it could be retrieved. The stopwatch continued running during tablet retrieval. When a cat dropped the tablet out of reach a second time, the stopwatch was stopped, the time and behavior recorded, and the tablet discarded. When an animal did not take the tablet in its mouth within 3 minutes of the offer, the tablet was withdrawn and the cat scored as not accepting the tablet. When an animal did not consume the entire tablet within 3 minutes of the offer, the cat was scored as not consuming the tablet.

### PRE-TREATMENT

Each cat's willingness to consume treats as determined by consumption of two commercially available cat treats was observed. 71.4% of the treats were consumed and all cats, with the exception of one cat, consumed at least one of the two treats at each offering indicating willingness by the majority of the cats to consume a treat. That one cat was never observed exhibiting any interest in the treats.

### STUDY TREATMENT

Frequency of tablet acceptance (took tablet in mouth) and tablet consumption (consumed entire tablet) are listed in Table 13. As can be seen by this information, BrewTech Dried Brewer's Yeast had much higher frequency of acceptance, 62.5%, and consumption, 51.8% than any other flavor. Sirius Stuff had the 2^{nd} highest frequency of acceptance and consumption. Vegetarian Beef was 3^{rd} for acceptance frequency and Artificial Chicken was 4^{th}; the two reserved order for consumption frequency.

**Table 13:**

| TABLET ACCEPTANCE AND CONSUMPTION FREQUENCY | | |
|---|---|---|
| FLAVOR | % ACCEPTED | % CONSUMED |
| BrewTech Dried Brewer's Yeast | 62.5% | 51.8% |
| Sirius Stuff | 42.9% | 25.0% |
| Artificial Chicken | 26.8% | 14.3% |
| Vegetarian Beef | 37.5% | 8.9% |
| Vegetarian Bacon | 23.2% | 0.0% |
| Lactose | 10.7% | 1.8% |
| Lactose + Tuna Oil | 33.9% | 1.8% |

### EXAMPLE 14

A palatable ANIPRYL (selegeline hydrochloride) (HCI) containing blend with 17% selegeline HCI, 6.67% PEG 8000 (polyethylene glycol having a molecular weight of 8000 daltons), 6.67% cross-linked povidone, 1.11% talc, about 50-60% microcrystalline cellulose as filler, about 5-20% flavor agent (artificial beef), 1.11% colloidal silicone dioxide, and 3% stearic acid was tabletted via direct compression.

### EXAMPLE 15

An icopezil containing granulation was prepared by a conventional aqueous high shear wet granulation process containing 1.31% Icopezil, 62.46% microcrystalline cellulose, 31.23% hydrous lactose, 2% sodium starch glycolate, 2% hydroxypropyl cellulose, and 1% magnesium stearate. The formulations were made into palatable tablets by extragranular addition of a flavor agent (brewers yeast or artificial beef) at levels of 5-20% and subsequently were compressed into tablets.

### EXAMPLE 16

Placebo tablets loaded with a known bittering agent (Bitrex) were prepared with and without artificial beef flavor. Results indicated that it was possible to overcome negative taste experiences in dogs with the addition of commercially available flavor agents (in this case 10-20% artificial beef).

The above preferred embodiments and Examples were given to illustrate the scope and spirit of the present invention. These embodiments and Examples will make apparent to those skilled in the art other embodiments and Examples. These other embodiments and Examples are within the contemplation of the present invention.

## Claims

1. A palatable pharmaceutical composition for oral administration to companion animals comprising a pharmaceutically effective amount of a pharmaceutically active agent in combination with a palatability improving agent and a pharmaceutically acceptable carrier, wherein the palatability improving agent is non-meat and non-fish derived, and is present in amounts sufficient to make the pharmaceutical composition palatable to the companion animal, provided that when the palatability improving agent is yeast, it is present in an amount ranging from about 2% to about 25% by weight of the palatable pharmaceutical composition.

2. The composition of claim 1 wherein the palatability improving agent is selected from the group consisting of artificial egg, artificial beef, artificial poultry, artificial fish, dairy based palatability improving agent and natural herbs and species, or a mixture thereof.

3. The pharmaceutical composition according to Claim 1 wherein the palatability improving agent is not yeast and is present in an amount ranging from about 1% to about 25% by weight of the pharmaceutical composition.

4. The composition of claim 1 wherein the palatability improving agent is yeast and is present in an amount ranging from about 2% to about 25% by weight of the pharmaceutical composition.

5. A palatable pharmaceutical composition for administration to companion animals comprising a pharmaceutical effective amount of a pharmaceutically active agent admixed with a palatability improving agent and in association with a pharmaceutical carrier, said palatability improving agent being a yeast or yeast hydrolysate, or a combination thereof, said yeast or yeast hydrolysate, or a combination thereof, being present in an amount ranging from abut 2% by weight to about 25% by weight of the pharmaceutical composition.

6. The pharmaceutical composition according to Claim 1 or Claim 5 in the form of a tablet.

7. A method of making a palatable pharmaceutical composition for oral administration to a companion animal which comprises mixing a pharmaceutically effective amount of a pharmaceutically active agent with an effective amount of a palatability improving agent and a pharmaceutical carrier and orally administering said product to the companion animal, the palatability improving agent being present in amounts sufficient to make the pharmaceutical palatable to the companion animal.

8. A method of making a pharmaceutical composition for oral administration to a companion animal palatable thereto which comprises admixing a pharmaceutically effective amount of the pharmaceutical with a palatability improving agent and a pharmaceutical carrier and orally administering said product to a companion animal, such palatability improving agent being a yeast or a yeast hydrolysate, said yeast or yeast hydrolysate being present in an amount ranging from about 2% to weight to about 25% by weight of the pharmaceutical composition.

9. A method for enhancing compliance with a therapeutic program for companion animals comprising administering to the animal a therapeutically effective amount of the pharmaceutical composition according to Claim 1 or Claim 5.

10. A palatable pharmaceutical composition for oral administration to companion animals comprising a pharmaceutically effective amount of a pharmaceutically active agent in combination with a palatability improving agent and a pharmaceutically acceptable carrier, wherein the palatability improving agent is non-meat, non-fish and non-yeast-derived, and is present in amounts sufficient to make the pharmaceutical composition palatable to the companion animal.
